# EUROPEAN PATENT APPLICATION

(11) **EP 4 644 352 A1**
(43) Date of publication of application: **05.11.2025**
(21) Application number: 25173729.2
(22) Date of filing: 30.04.2025
(51) Int. Cl.: C05F 11/08, C05G 5/18, C12N 1/20, A01N 63/22, A01N 63/27, A01N 63/38, C12N 1/14, C12N 1/16, C12N 11/00

(54) **PROCEDURE FOR MAKING A BIOFERTILISER COMPOSITION**

(30) Priority: 03.05.2024 IT 202400009985
(71) Applicant: Smileybee Ltd, Nicosia, 2018 (CY)
(72) Inventor: SUBRAYALU, KARTHIKAYALU, Chennai (IN)
(74) Representative: Lunati & Mazzoni S.r.L.

(57) **Abstract**

Procedure for making a biofertiliser composition (1) comprising:
a first growth step wherein at least one first microbiological culture (2) is produced;
a first preparation step wherein a culture medium (3) is produced; a first mixing step wherein the first culture (2) is mixed with the culture medium (3) to obtain a second microbiological culture (4); a second growth step wherein the second culture (4) is subjected to fermentation to obtain a third microbiological culture (5); a separation step wherein from the third culture (5) a fourth microbiological culture (6) and a residual culture medium (30) are separated; a synthesising step wherein a gel (8) is synthesised; and a second mixing step wherein the gel (8) is mixed with the fourth culture (6) to obtain the composition (1).

## Description

The present invention relates to a procedure for making a biofertiliser composition of the type specified in the preamble of the first claim.

The object of the present invention is a procedure for making a biofertiliser composition which finds application mainly, but not exclusively, in the field of the chemical industry and agriculture.

Fertilisers are technical means, which are typically used in agriculture and gardening, that allow the fertility of the soil to be improved.

They can have different effects on the soil, depending on their own composition and the composition of the soil. In particular, they can be classified based on the type of improvement they confer to the soil.

Among the types of known fertilisers, fertilisers, soil improvers and correctives can be distinguished.

Fertilisers perform the function of enriching the soil with one or more nutrient elements.

Soil improvers allow the improvement of the physical properties of the soil by modifying its structure and/or texture.

Correctives perform the function of modifying the reaction of abnormal soils by shifting the pH towards neutrality.

A particular class of fertilisers is that of biofertilisers. They are systems for the production of nutrients in which biological proedures are involved. In fact, they can include selective microorganisms such as bacteria, fungi and algae, which are capable of fixing atmospheric nitrogen or converting soluble phosphate and potassium in the soil into forms available to plants.

Biofertilisers are advantageous since they provide an economical, renewable and ecological source of nutrients. They also allow the maintenance of soil fertility and sustainability over the long term.

One of the functions that biofertilisers can perform is to help the plant to access the nutrients present in the surrounding environments.

Carrier-based biofertilisers allow an effective preparation of the inoculum of biofertilisers.

The known technique described includes some important drawbacks.

In particular, carrier-based biofertilisers have limitations such as a low shelf-life, poor survival under adverse conditions, a high degree of contamination and inconsistent field performance.

In this situation, the technical task underlying the present invention is to devise a procedure for making a biofertiliser composition capable of substantially overcoming at least part of the aforesaid drawbacks.

Within the scope of said technical task, it is an important object of the invention to achieve a procedure for making a biofertiliser composition that allows the production of biofertilisers capable of being stored for long periods before use.

Another important object of the invention is to achieve a procedure for making a biofertiliser composition that allows the production of biofertilisers capable of better withstanding adverse environmental conditions.

A further object of the invention is to achieve a procedure for making a biofertiliser composition that allows the production of biofertilisers capable of operating with greater efficiency.

The technical task and the objects specified are achieved by a procedure for making a biofertiliser composition as claimed in the annexed claim 1.

Preferred technical solutions are highlighted in the dependent claims.

The features and advantages of the invention are clarified below by the detailed description of preferred embodiments of the invention, with reference to the accompanying drawings, in which:

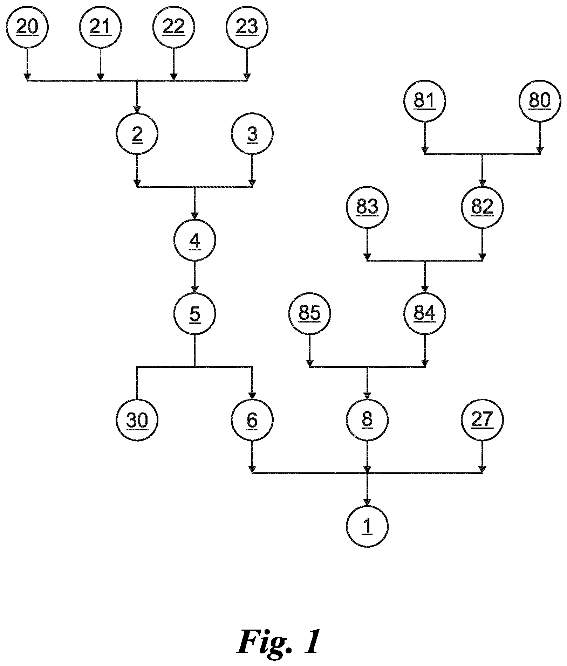

**Figure 1** shows a first diagram of a procedure for making a biofertiliser composition according to the invention;

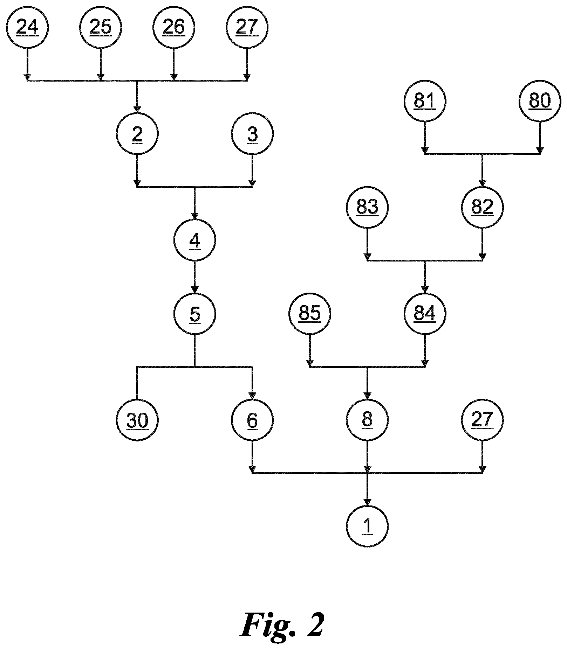

**Figure 2** shows a second diagram of a procedure for making a biofertiliser composition according to the invention;

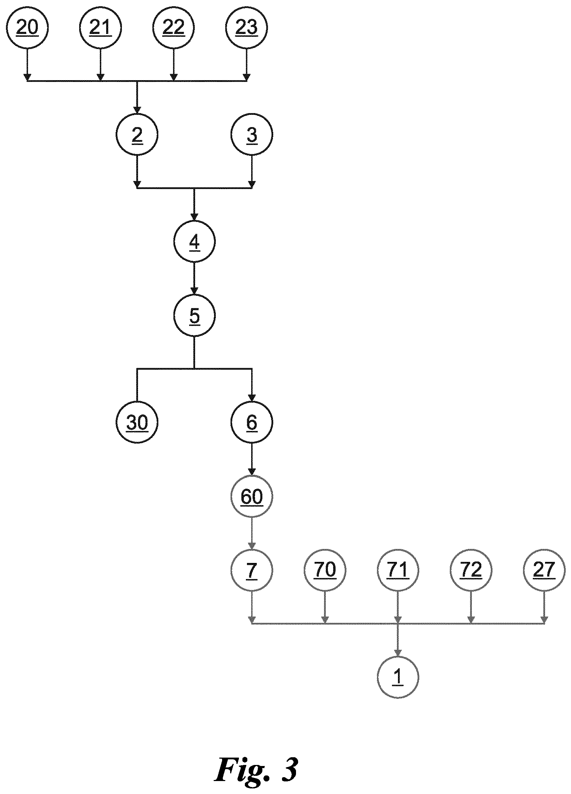

**Figure 3** shows a third diagram of a procedure for making a biofertiliser composition according to the invention; and

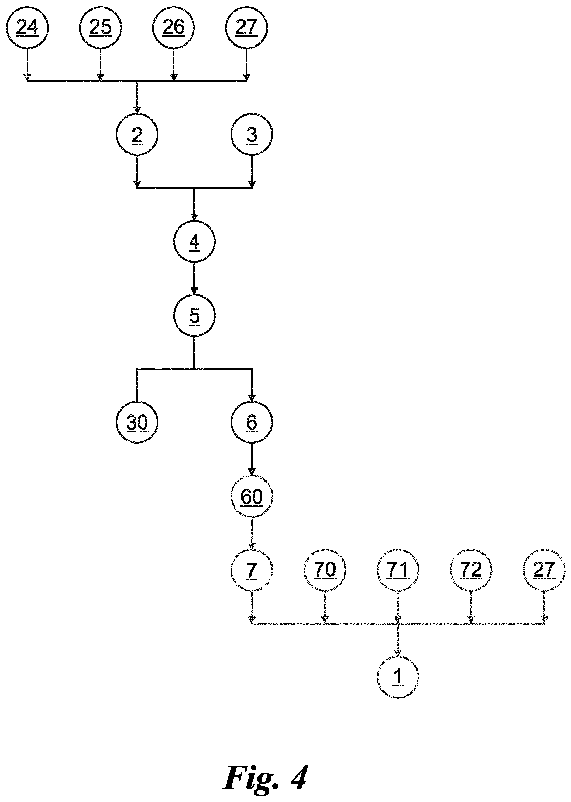

**Figure 4** shows a fourth diagram of a procedure for making a biofertiliser composition according to the invention.

In the present document, measurements, values, shapes and geometric references (such as perpendicularity and parallelism), when associated with words such as "about" or other similar terms such as "approximately" or "substantially", are to be understood as subject to measurement errors or inaccuracies due to production and/or manufacturing errors and, above all, subject to a slight deviation from the value, measurement, shape or geometric reference with which they are associated. For example, such terms, if associated with a value, preferably indicate a deviation not greater than 10% of the value itself.

Furthermore, when used, terms such as "first", "second", "upper", "lower", "main" and "secondary" do not necessarily identify an order, a priority of relationship or relative position, but can simply be used to more clearly distinguish different components from one another.

Unless otherwise specified, as results from the following discussions, it is considered that terms such as "processing", "computing", "determination", "calculation", or the like, refer to the action and/or processes of a computer or similar electronic computing device that manipulates and/or transforms data represented as physical quantities, such as electronic quantities of registers of a computer system and/or memories into other data similarly represented as physical quantities within computer systems, registers or other devices for storing, transmitting or displaying information.

The measurements and data reported in the present text are to be considered, unless otherwise indicated, as carried out in International Standard Atmosphere ICAO (ISO 2533:1975).

With reference to the Figures, the composition made by the procedure for making according to the invention is globally denoted by the number 1.

The invention comprises a new procedure for making a biofertiliser composition 1. It is a composition that can be used at least to increase the fertility of a soil.

The composition 1 is preferably made by the procedure for making according to the invention.

The procedure for making the biofertiliser composition 1 preferably comprises at least one sterilisation step. In this step, the containers used for the steps of the procedure are subjected to sterilisation. The latter allows the procedure to be carried out in such a way as to prevent any contamination that could lead to an alteration of the composition 1.

In detail, the sterilisation step can preferably be carried out at a temperature of at least 120°C. More preferably, the sterilisation step can be carried out at a temperature of at least 121°C. Furthermore, the sterilisation step can be carried out at a pressure of at least 1.02•10⁵ Pa. More preferably, sterilisation can be carried out at a pressure of 1.03•10⁵ Pa. The sterilisation step can last at least 15 minutes. In this way, an efficient sterilisation procedure can be achieved.

The procedure comprises at least a first growth step. In this step, at least one first microbiological culture 2 is produced. The first culture 2 preferably comprises at least one among nitrogen-fixing bacteria 20, phosphorus solubilising bacteria 21, potassium-mobilising bacteria 22 and Trichoderma viride 23. The nitrogen-fixing bacteria 20 are bacteria that advantageously transform the nitrogen present in the soil into forms available for uptake by plants. Similarly, the phosphorus solubilising bacteria 21 allow phosphorus to be brought into the soil solution so that it becomes available to plants.

The potassium-mobilising bacteria 22 have the advantage of transporting potassium present in the soil to plants and thus making it available for absorption by the latter. Trichoderma viride 23 are a type of fungi that mainly perform an antiparasitic action. In particular, Trichoderma viride 23 advantageously allow the elimination of fungi and bacteria present in the rhizosphere. Furthermore, they can also stimulate plants to enhance their defences against parasitic organisms. Therefore, they can effectively perform a synergistic action in the formulation of the biofertiliser, since they do not directly perform a nutrient transport function, but favour plant growth by reducing risks to its development.

The first culture 2 preferably comprises a quantity of nitrogen-fixing bacteria 20 of at least 10¹⁰ CFU (colony forming unit)/mL. More preferably, 10¹¹ CFU/mL. The first culture 2 may comprise a quantity of phosphorus solubilising bacteria 21 of at least 10¹⁰ CFU/mL. More preferably, this quantity may be 10¹¹ CFU/mL. The first culture 2 may preferably comprise a quantity of potassium-mobilising bacteria 22 of at least 10¹⁰ CFU/mL. More preferably, it may be 10¹¹ CFU/mL. Furthermore, the first culture 2 may comprise a quantity of Trichoderma viride 23 of at least 10⁸ CFU/mL. More preferably, the quantity of Trichoderma viride 23 may be 10⁹ CFU/mL.

In the first growth step, the first culture 2 may comprise all the microorganisms previously described in the reported quantities.

Furthermore, the first culture 2 may preferably comprise at least one among Acetobacter spp 24, Pseudomonas flourescens 25, Bacillus subtilis 26, Trichoderma viride 23, Vesicular Arbuscular Mycorrhiza 27.

The Acetobacter spp 24 can perform a function of regulating the pH of the soil. The Pseudomonas fluorescens 25 perform a function of protecting plant roots by preventing diseases due to bacteria and fungi present in the soil.

Vesicular Arbuscular Mycorrhiza 27 allow nutrients, water and protection from pathogenic microorganisms to be supplied to plants.

The first culture 2 preferably comprises a quantity of Acetobacter spp 24 of at least 10⁹ CFU. More preferably, 10¹⁰ CFU/mL. The first culture 2 may comprise a quantity of Pseudomonas flourescens 25 of at least 10⁹ CFU/mL. More preferably, this quantity may be 10¹⁰ CFU/mL. The first culture 2 may preferably comprise a quantity of Bacillus subtilis 26 of at least 10⁹ CFU/mL. More preferably, it may be 10¹⁰ CFU/mL. Furthermore, the first culture 2 may comprise Trichoderma viride 23 for at least 0.1%. More preferably, for at least 0.5%. In the first culture 2, a percentage of Vesicular Arbuscular Mycorrhiza 27 of at least 1% may be present. More preferably, the percentage is 2%.

In a possible embodiment of the first growth step, the first culture 2 may comprise all the microorganisms previously described: Acetobacter spp 24, Pseudomonas flourescens 25, Bacillus subtilis 26, Trichoderma viride 23, Vesicular Arbuscular Mycorrhiza 27, in the reported quantities.

The procedure comprises a first preparation step in which a culture medium 3 is produced. In this step, the culture medium 3 is preferably conditioned to a temperature of 25°C. More preferably, it is conditioned to a temperature of at least 30°C. In particular, these temperatures are reached if the first culture 2 comprises at least one of nitrogen-fixing bacteria 20, Trichoderma viride 23 and Acetobacter spp 24.

Similarly, the culture medium 3 is preferably conditioned to a temperature of at least 30°C. More preferably, it is conditioned to 37°C. In particular, these temperatures are reached if the first culture 2 comprises at least one of phosphorus solubilising bacteria 21, potassium-mobilising bacteria 22, Pseudomonas flourescens 25 and Bacillus subtilis 26. These temperatures of the culture medium 3 make the subsequent steps of the procedure more effective.

Each of the microorganisms previously described may be subjected to the first growth step separately as separate first cultures 2, which may subsequently be combined.

The procedure comprises a first mixing step. In this step, the first culture 2 is mixed with the culture medium 3 to obtain a second microbiological culture 4. The latter therefore comprises the second culture 2 mixed with the culture medium 3.

The culture medium 3 is preferably conditioned as previously described. The mixing step ensures that the second culture 4 obtained will more probably undergo further growth.

The procedure comprises a second growth step in which the second culture 4 is subjected to fermentation to obtain a third microbiological culture 5.

The latter comprises microorganisms in greater quantities than the second culture 4, as a result of the growth procedure.

Subsequently, the procedure may comprise a separation step, in which from the third culture 5 a fourth microbiological culture 6 and a residual culture medium 30 are separated. The fourth culture 6 corresponds to the third culture 5 deprived of at least part of the residual culture medium 30. The latter corresponds to the culture medium still present in the third culture 5 at the end of the second growth step.

In this step, the third culture 5 is preferably subjected to centrifugation at a speed of 10000 rpm and at a flow rate of 100 L/hr. In this way, the fourth culture 6 and the residual culture medium 30 can be separated and collected.

The residual culture medium 30 can be subjected to a recovery step, passing through a water treatment stage, to recover water from the residual culture medium 30. In the recovery step, the solid waste present in the residual culture medium 30 and the wastewater can be disposed of. The treated water can be reused in the initial steps of a new procedure.

The procedure advantageously comprises a synthesising step. In this step, a gel 8 is synthesised. The gel 8 has properties whereby it is not toxic to the microorganisms.

In detail, the synthesising step may preferably comprise a first sub-step. In this sub-step, chitosan 80 is added and mixed with an acetic acid solution 81.

Preferably, an amount between 2 g and 8 g of chitosan 80 is added and mixed with the acetic acid solution 81. More preferably, the amount of added chitosan 80 is between 3 g and 7 g. Even more preferably, the amount of added chitosan 80 is between 4 g and 6 g. The acetic acid 81 preferably has a concentration between 0.5% and 1.5%.

In this way, a chitosan gel 82 is synthesised.

The chitosan gel 82 is advantageous due to its non-toxicity towards microorganisms. It preferably has a chitosan 80 concentration between 2% and 8%. More preferably, it is between 3% and 7%. Even more preferably, it is between 4% and 6%.

The synthesising step may preferably comprise a second sub-step. In this sub-step, cross-linked cellulose 83 is added to the chitosan gel 82. In this way, an added chitosan gel 84 is obtained. The latter has a structure more resistant than the non-added chitosan gel 82.

In detail, in the second step, the cross-linked cellulose 83 is preferably added in such amounts as to be present at a concentration of at least 0.5% in the added chitosan gel 84.

The synthesising step preferably comprises a third sub-step. The third sub-step allows the gel 8 to be obtained. In this sub-step, xanthan gum 85 is added to the added chitosan gel 84. In detail, in this sub-step, xanthan gum 85 is added to chitosan gel 84 in such an amount as to be present at a concentration of 1% in the gel 8.

The procedure comprises a mixing step in which the gel 8 is mixed with the fourth culture 6. In this way, the composition 1 is obtained.

In detail, in the second mixing step, Vesicular Arbuscular Mycorrhiza 27 may preferably be added and mixed with the fourth culture 6 and the gel 8. It may be added in quantities such as to have 10000 spores/g. Furthermore, the gel 8, subsequent to the second mixing step, is present in a concentration between 1% and 3% in the composition 1.

At the end of the making procedure, the biofertiliser composition 1 is obtained. The procedure may preferably comprise, as a replacement for the synthesising step and the second mixing step, a freezing step.

In this step, the fourth culture 6 is conditioned at cryogenic temperatures. In this way, a fourth processed culture 60 is obtained. In this step, the fourth culture 6 is preferably conditioned at a temperature lower than -30°C. More preferably, the fourth culture 6 is conditioned at a temperature lower than -40°C. The freezing step may preferably last at least 40 hours. More preferably, it may last at least 50 hours. Furthermore, this step may preferably be conducted at a pressure lower than 500 mTorr. More preferably, it may be conducted at a pressure lower than 400 mTorr. Even more preferably, it may be conducted at a pressure lower than 300 mTorr. The procedure may preferably comprise a pulverisation step. In this step, the frozen fourth culture 60 is subjected to pulverisation. In this way, a fourth pulverised culture 7 is obtained. The frozen fourth culture 60 may be reduced to powder by crushing. The procedure may preferably comprise a third mixing step. In this step, the fourth pulverised culture 7 obtained after crushing is added and mixed at least with dextrose 70, starch 71, sodium chloride 72 and Vesicular Arbuscular Mycorrhiza 27. In this way, the composition 1 is advantageously obtained.

In detail, in the third mixing step, additions are made in such a way that the composition 1 preferably comprises a percentage amount between 1% and 3% of the fourth pulverised culture 7. More preferably, it comprises a percentage between 1.5% and 2.5%. Furthermore, the composition 1 preferably comprises a percentage of at least 90% of dextrose 70. The composition 1 preferably comprises a percentage between 4% and 8% of starch 71. More preferably, between 5% and 7%. The composition 1 preferably comprises a percentage of sodium chloride 72 between 0.05% and 0.15%. Furthermore, the composition 1 preferably comprises a percentage of Vesicular Arbuscular Mycorrhiza 27 between 1% and 3%. More preferably, between 1.5% and 2.5%.

In this way, a composition 1 is obtained in which the microorganisms are advantageously incorporated within the structure formed by starch 71 and dextrose 70. This structure allows a protective effect on the microorganisms present inside the fourth pulverised culture 7.

The making procedure according to the invention achieves important advantages. Indeed, it allows making a biofertiliser that does not deteriorate rapidly and can be stored for long periods before use. This characteristic of composition 1 is due to the gel incorporating the microorganisms contained in the microbiological cultures. In fact, it ensures favourable conditions that allow the microorganisms to be maintained alive for longer periods thanks to favourable conditions.

Furthermore, the presence of the gel ensures that the effectiveness of the microorganisms is improved and less dependent on the specific soil conditions. The microorganisms, therefore, by resisting adverse environmental conditions, can better tolerate transport conditions and changes in environmental conditions. Moreover, the microorganisms are immediately available to perform their fertilising action and do not require an activation period to reach maximum efficiency. Another important advantage of the biofertiliser made by the procedure is the effectiveness of its fertilising action.

The gel can perform a symbiotic action with the plants that increases the effectiveness of the biofertiliser activity.

Indeed, the gel facilitates the absorption of nitrogen by plants with high efficiency. The gel also makes the transport of nutrients itself more effective.

An advantage of the composition obtained through the pulverisation procedure lies in the advantage of retaining the microorganisms within the starch structure, acting similarly to the gel, with similar advantages.

The invention is susceptible to variants falling within the scope of the inventive concept defined by the claims. Within such scope, all details may be replaced by equivalent elements and the materials, shapes and dimensions may be any.

## Claims

1. Procedure for making a biofertiliser composition (1) comprising:
- a first growth step wherein at least one first microbiological culture (2) is produced;
- a first preparation step wherein a culture medium (3) is produced;
- a first mixing step wherein said first culture (2) is mixed with said culture medium (3) to obtain a second microbiological culture (4);
- a second growth step wherein said second culture (4) is subjected to fermentation to obtain a third microbiological culture (5);
- a separation step wherein a fourth microbiological culture (6) and a residual culture medium (30) are separated from said third culture (5);
and **characterised in that** it further comprises:
- a synthesising step wherein a gel (8) is synthesised,
- a second mixing step wherein said gel (8) is mixed with said fourth culture (6) to obtain said composition (1).

2. Procedure according to claim 1, wherein said first culture (2) comprises at least one of:
- nitrogen-fixing bacteria (20);
- phosphorus solubilising bacteria (21);
- potassium-mobilising bacteria (22);
- Trichoderma viride (23).

3. Procedure according to the previous claim, wherein said first culture (2) comprises an amount of said nitrogen-fixing bacteria (20) of at least 10¹⁰ CFU (colony forming unit)/mL, a quantity of said phosphorus solubilising bacteria (21) of at least 10¹⁰ CFU/mL (Colony Forming Unit/mL), a quantity of said potassium-mobilising bacteria (22) of at least 10¹⁰ CFU/mL, and a quantity of Trichoderma viride (23) of at least 10⁸ CFU/mL.

4. Procedure according to any one of claims 1-3, wherein said first culture (2) comprises at least one of:
- Acetobacter spp (24);
- Pseudomonas flourescens (25);
- Bacillus subtilis (26);
- Trichoderma viride (23);
- Vesicular Arbuscular Mycorrhiza (27).

5. Procedure according to the previous claim, wherein said first culture (2) comprises an amount of said Acetobacter spp (24) of at least 10⁹ CFU/mL, a quantity of said Pseudomonas flourescens (25) of at least 10⁹ CFU/mL, a quantity of said Bacillus subtilis (26) of at least 10⁹ CFU/mL, a percentage of said Trichoderma viride (23) of at least 0.1%, and a percentage of said Vesicular Arbuscular Mycorrhiza (27) of at least 1%.

6. Procedure according to any one of the preceding claims, comprising at least one sterilisation step wherein the containers used for the steps of said procedure are subjected to sterilisation at a temperature of at least 120°C and a pressure of at least 1.02•10⁵ Pa for at least 15 minutes.

7. Procedure according to at least claims 2 and 4, wherein prior to said first mixing step said culture medium (3) is conditioned to a temperature of 30°C if said first culture (2) comprises at least one of said nitrogen-fixing bacteria (20), said Trichoderma viride (23) and said Acetobacter spp (24).

8. Procedure according to at least claim 4, wherein prior to said first mixing step said culture medium (3) is conditioned to a temperature of 37°C if said first culture (2) comprises at least one of said phosphorus solubilising bacteria (21), said potassium-mobilising bacteria (22), said Pseudomonas flourescens (25) and said Bacillus subtilis (26).

9. Procedure according to any one of the preceding claims, wherein in said separation step said third culture (5) is subjected to centrifugation at a speed of 10000 rpm and a flow rate of 100 L/hr.

10. Procedure according to any one of the preceding claims, wherein said synthesising step comprises:
- a first sub-step wherein chitosan (80) is added and mixed with an acetic acid solution (81) to synthesise a chitosan gel (82);
- a second sub-step wherein cross-linked cellulose (83) is added to said chitosan gel (82) to obtain an added chitosan gel (84);
- a third sub-step wherein xanthan gum (85) is added to said added chitosan gel (84) to obtain said gel (8).

11. Procedure according to the previous claim, wherein in said first sub-step an amount between 2 g and 8 g of said chitosan (80) is added and mixed with said acetic acid solution (81) having a concentration between 0% and 1.5%, and said chitosan gel (82) has a concentration of said chitosan (80) between 2% and 8%.

12. Procedure according to any one of claims 11-12, wherein:
- in said second sub-step said cross-linked cellulose (83) is added in such an amount as to be present at a concentration of at least 0.5% in said added chitosan gel (84); and
- in said third sub-step said xanthan gum (85) is added to said chitosan gel (84) in such an amount as to be present at a concentration of 1% in said gel (8).

13. Procedure according to any one of the preceding claims, wherein in said second mixing step said fourth culture (6) and said gel (8) are added and mixed with said Vesicular Arbuscular Mycorrhiza (27) in such an amount as to have 10000 spores/g and, subsequent to said second mixing step, are present in a concentration between 1% and 3% in said composition (1).

14. Procedure according to any one of claims 1-10, wherein said synthesising step and said second mixing step are replaced by:
- a freezing step wherein said fourth culture (6) is conditioned at cryogenic temperatures to obtain a fourth processed culture (60);
- a pulverisation step wherein said fourth frozen culture (60) is subjected to pulverisation to obtain a fourth pulverised culture (7);
- a third mixing step wherein said fourth pulverised culture (7) is added and mixed at least with dextrose (70), starch (71), sodium chloride (72) and said Vesicular Arbuscular Mycorrhiza (27) to obtain said composition (1).

15. Procedure according to the previous claim, wherein in said freezing step said fourth culture (6) is conditioned at a temperature lower than -30°C and for a duration of at least 40 hours at a pressure lower than 500 mTorr.

16. Procedure according to any one of claims 15-16, wherein said composition (1) comprises the following percentage amounts:
- between 1% and 3% of said fourth pulverised culture (7);
- at least 90% of said dextrose (70);
- between 4% and 8% of said starch (71);
- between 0.05% and 0.15% of said sodium chloride (72);
- between 1% and 3% of said Vesicular Arbuscular Mycorrhiza (27).

17. Biofertiliser composition (1) made by the making procedure according to any one of the preceding claims.
